# EUROPEAN PATENT APPLICATION

(11) **EP 4 682 254 A1**
(43) Date of publication of application: **21.01.2026**
(21) Application number: 24771216.9
(22) Date of filing: 15.03.2024
(51) Int. Cl.: C12N 9/52, C07K 14/33, C07K 1/16, C07K 1/18

(54) **IMPROVED METHOD OF PURIFYING BOTULINUM TOXIN USING MULTIMODAL CHROMATOGRAPHY**

(30) Priority: 16.03.2023 KR 20230034548
(71) Applicant: Daewoong Co., Ltd., Seongnam-si, Gyeonggi-do 13211 (KR)
(72) Inventor: SO, Yong In, Gyeonggi-do 18612 (KR); LEE, Min A, Gyeonggi-do 18430 (KR); KIM, Sung Soo, Gyeonggi-do 18601 (KR); JEON, Sang Eun, Seoul 02798 (KR); PARK, Ji Seong, Gyeonggi-do 18374 (KR); CHOO, Seung Jung, Gyeonggi-do 18500 (KR); KIM, Se Mi, Gyeonggi-do 18599 (KR); KANG, Yu Jin, Gyeonggi-do 18486 (KR); KIM, Kyoung Yun, Jeonbuk-do 54868 (KR)
(74) Representative: Mewburn Ellis LLP
(86) International application number: PCT/KR2024/003283
(87) International publication number: WO 2024/191217

(57) **Abstract**

Disclosed is a method of purifying botulinum toxin including purifying botulinum toxin from a sample containing botulinum toxin using multimodal chromatography and purifying the botulinum toxin using cation exchange chromatography. The method of purifying botulinum toxin enables purification of botulinum toxin with a high purity of 99% or more and high yield, and thus is useful for botulinum toxin production.

## Description

### BACKGROUND OF THE INVENTION

### Field of the Invention

The present invention relates to a method of purifying botulinum toxin, and more specifically, to a method of purifying botulinum toxin including purifying botulinum toxin using multimodal chromatography and purifying the toxin using cation exchange chromatography.

### Description of the Related Art

Various strains of the genus *Clostridium* that secrete neurotoxins have been discovered since the 1890s, and the toxins secreted by these strains have been characterized over the last 70 years.

The neurotoxin derived from the *Clostridium* genus strains, that is, botulinum toxin, is classified into seven types, namely A to G, according to the serological characteristics thereof. Each toxin has a toxin protein of about 150 kDa, which is naturally bound with non-toxic proteins to form a complex. The medium complex (300 kDa) consists of a toxin protein and a non-toxic non-hemagglutinin protein, and the large complex (450 kDa) and the large-large (900 kDa) complex have a structure in which an intermediate complex is bound to hemagglutinin (Sugiyama, H, Microbiol Rev, 44:419, 1980). These non-toxic non-hemagglutinin proteins are known to function to protect toxins from low pH and various proteases in the intestine.

The toxin is synthesized as a single polypeptide having a molecular weight of about 150 kDa in cells and is then cleaved into a light chain (L, molecular weight: 50 kDa) and a heavy chain (H, molecular weight: 100 kDa) at a position one third from the N-terminus by the action of an intracellular protease or treatment with an artificial enzyme such as trypsin. The toxicity of the cleaved toxin is greatly increased compared to that of a single polypeptide. The two units are linked to each other by disulfide bonds and have different functions. The heavy chain binds to the receptor of a target cell and reacts with the biological membrane at a low pH (pH of 4) to form a channel (Mantecucco, C. et al., TIBS, 18:324, 1993), whereas the light chain has pharmacological activity and thus imparts permeability to cells in the presence of a detergent or impedes the secretion of neurotransmitters when injected into cells by electroporation.

The toxin inhibits the exocytosis of acetylcholine at the cholinergic presynapse of a neuromuscular junction to thereby cause asthenia. Treatment with a very small amount of the toxin exhibits toxicity, suggesting that the toxin has enzymatic activity.

According to a recent report, the toxin has metallopeptidase activity, and its substrate is composed of synaptobrevin, syntaxin, a synaptosomal-associated protein of 25 kDa (SNAP25) and the like, which are the unit proteins of an exocytosis machinery complex. Each type of toxin uses one of the above-described three proteins as its substrate, and it is known that type B, D, F and G toxins cleave synaptobrevin at a specific site, type A and E toxins cleave SNAP25 at a specific site, and type C cleaves syntaxin at a specific site.

Particularly, type A botulinum toxin is known to be soluble in a dilute aqueous solution at a pH of 4.0-6.8. It is known that a stable non-toxic protein is separated from the neurotoxin at a pH of about 7 or higher, and as a result, the toxicity is gradually lost. Particularly, it is known that the toxicity decreases as pH and temperature increase.

Botulinum toxin is fatal to humans even in small amounts and is easy to produce in large amounts. Thus, it is considered one of the four major biological terror weapons along with *Bacillus anthracis, Yersinia pestis* and smallpox virus. However, it was found that, when type A botulinum toxin is injected at a dose that does not systematically affect the human body, it can paralyze local muscle at the injection site. Based on this characteristic, type A botulinum toxin can be used in a wide range of applications, including wrinkle removal agents, agents for treating spastic hemiplegia and cerebral palsy, and the like. Thus, the demand for type A botulinum toxin has increased, and studies on methods of producing botulinum toxin to satisfy the demand are being actively conducted.

Botulinum toxin for clinical use is generally isolated from cell culture and various purification methods are used therefor.

Acid precipitation, salt precipitation, and chromatography were used for conventional botulinum toxin production.

For example, Japanese Patent Laid-Open No. 1994-192296 discloses a method of producing crystalline botulinum type A toxin including culturing Clostridium botulinum strains and then performing acid precipitation, extraction, addition of a nuclease, and crystallization. In addition, U.S. Patent No. 5696077 discloses a method of producing botulinum type B toxin including culturing *Clostridium botulinum* strains and performing acid precipitation, extraction, ion exchange chromatography, gel filtration chromatography, and crystallization.

Meanwhile, Simpson *et al.* produced botulinum type B toxin by purification of botulinum neurotoxin using gravity flow chromatography, HPLC, capture using an affinity resin, size exclusion chromatography, and ion (anion and cation) exchange chromatography using two different ion exchange columns (Method in Enzymology, 165:76, 1988), and Wang et *al.* used precipitation and ion chromatography to purify botulinum toxin type A (Dermatol Las Faci Cosm Surg., 2002:58, 2002).

In addition, US Patent No. 6818409 discloses the use of ion exchange and lactose columns to purify botulinum toxin, and US Patent No. 7452697 discloses a method of producing botulinum type A toxin using ion exchange chromatography and hydrophobic chromatography. Korean Patent Publication No. 2009-0091501 discloses a method of purifying botulinum toxin using acid precipitation and anion exchange chromatography, and US Patent Publication No. 2013-0156756 discloses a method of purifying botulinum toxin using anion exchange chromatography and cation exchange chromatography.

Recently, nucleic acids and animal-derived products that may be incorporated as impurities during the purification process of botulinum toxin have been continuously reported to cause serious side effects. Therefore, there is a continuous need for a method of purifying botulinum toxin with higher purity. Currently, methods of purifying botulinum toxin commonly used in the art yield only a purity of about 95 to 96%. Despite the need for a high-purity purification process, purity and yield vary greatly even depending on simple change in the purification sequence, thus making it difficult to predict the results of purification. Even when the purity is increased by about 1%, the yield greatly decreases, making it economically inefficient. Therefore, there is a difficulty in developing a method of purifying high-purity botulinum toxin.

Under this technical background, as a result of extensive efforts to develop a method of purifying botulinum toxin that is capable of obtaining botulinum toxin in high yield while maintaining a high purity of 99% or higher, the present inventors found that, when size exclusion chromatography is introduced in multimode or sequence into the purification, botulinum toxin can be purified with high purity of 99% or higher, low aggregation of 0.5% or less, and a higher yield than the prior art. Based on this finding, the present invention has been completed.

### SUMMARY OF THE INVENTION

Therefore, the present invention has been made in view of the above problems, and it is one object of the present invention to provide a method of purifying botulinum toxin with high purity and high yield.

In accordance with one aspect of the present invention, the above and other objects can be accomplished by the provision of a method of purifying botulinum toxin including (a) purifying botulinum toxin from a sample containing botulinum toxin using anion exchange chromatography (AEX), hydrophobic interaction chromatography (HIC), and size exclusion chromatography (SEC), and (b) purifying the botulinum toxin using cation exchange chromatography.

### BRIEF DESCRIPTION OF THE DRAWINGS

The above and other objects, features and other advantages of the present invention will be more clearly understood from the following detailed description taken in conjunction with the accompanying drawings, in which:

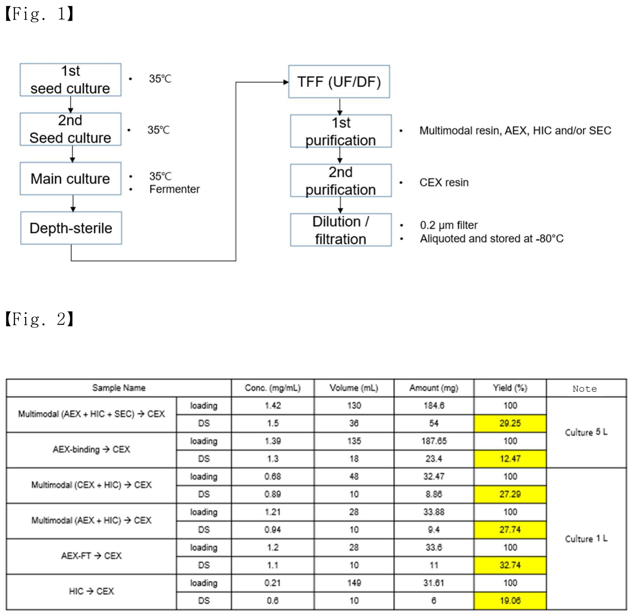

FIG. 1 is a schematical diagram illustrating the overall production process of botulinum toxin used in embodiments of the present invention, wherein 1st purification includes 1st purification processes used in Examples 3-1 to 3-8 and 5-1, 2nd purification includes 2nd purification processes (CEX) used in Examples 4-1 and 5-2, and in Examples 3-7, 1st purification includes performing sequentially multimodal chromatography (AEX+HIC) and SEC;

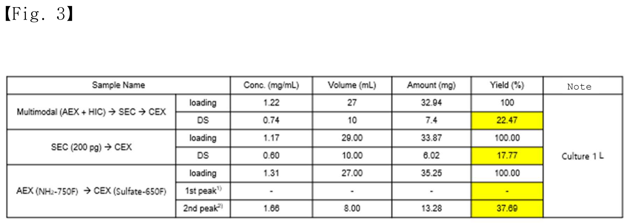

FIGS. 2 and 3 show the yields of toxins obtained after purification of the toxins using various combinations of chromatography including the purification process of the present invention;

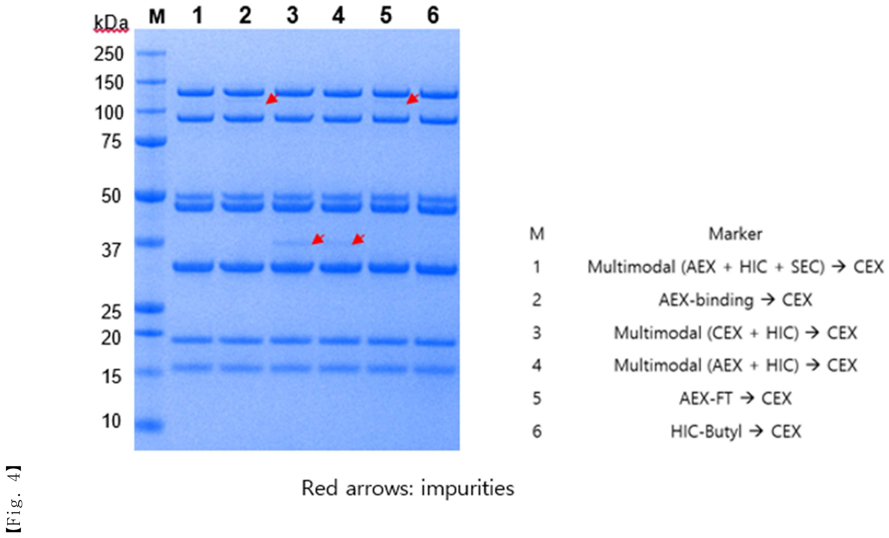

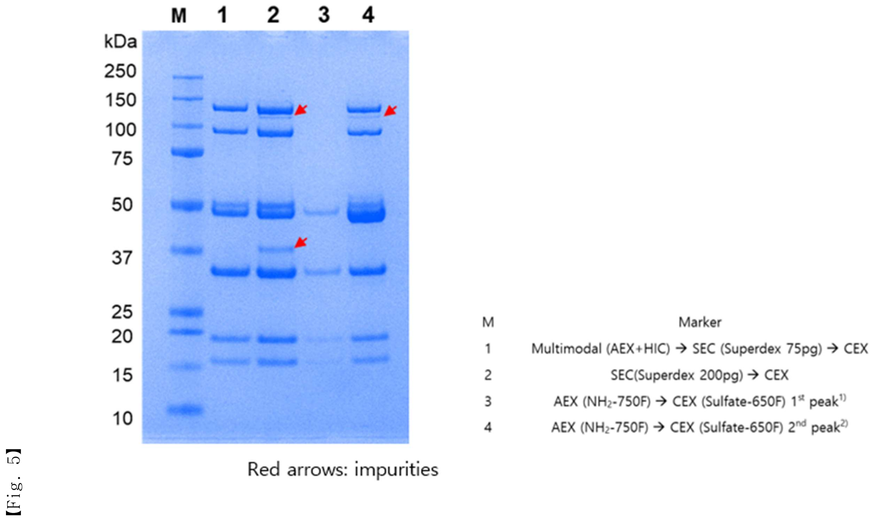

FIGS. 4 and 5 show the results of identification of the presence of impurities through SDS-PAGE after purification of toxins using various combinations of chromatography including the purification process of the present invention, wherein Red arrows represent impurity bands, footnote 1) represents a peak containing botulinum toxin described in the prior literature, and footnote 2) represents a peak containing toxin identified in the example; and

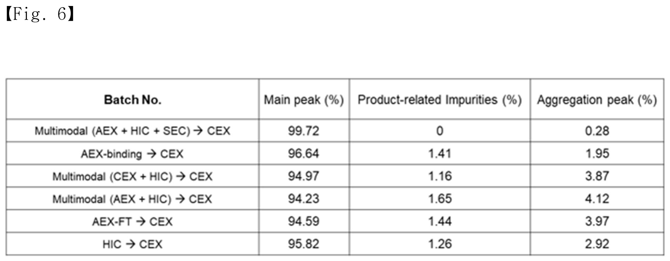

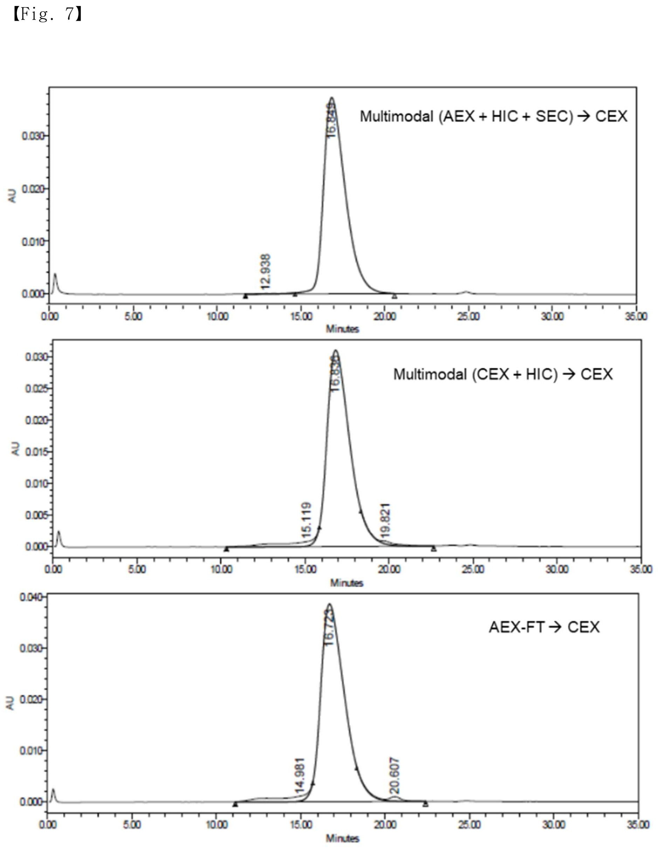

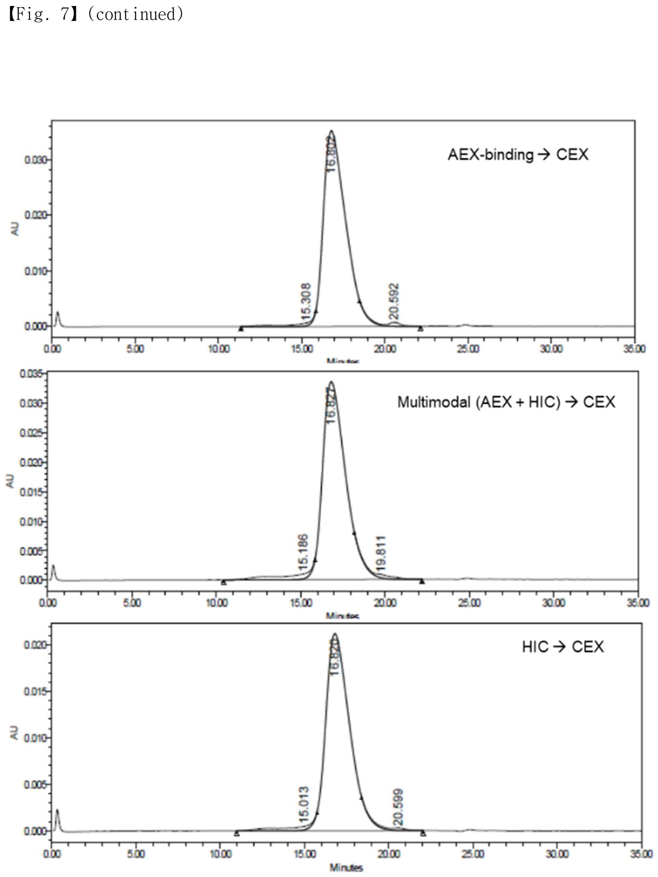

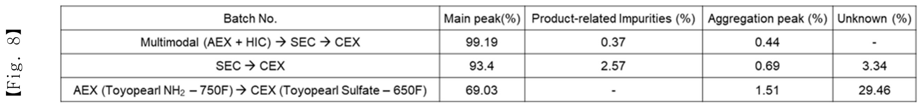

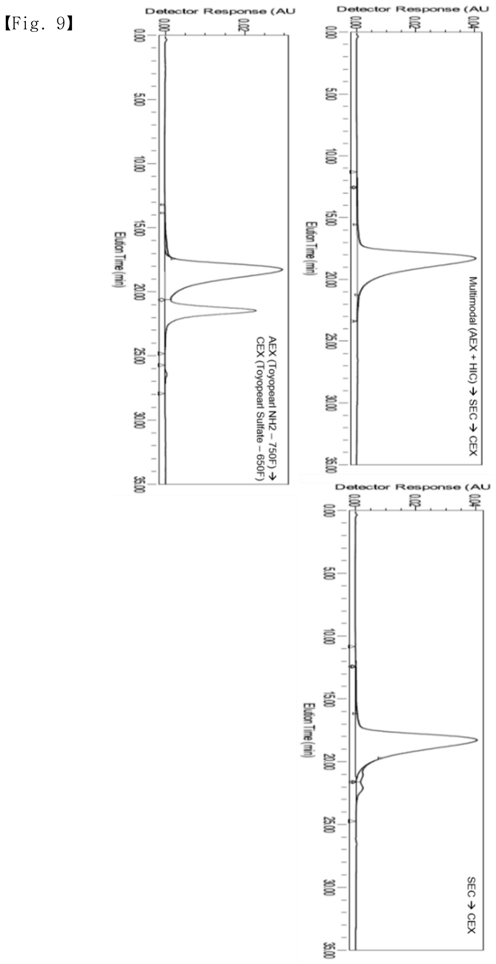

FIGS. 6 to 9 show purities, impurity peaks, and aggregation peaks measured through SDS-PAGE after purification of toxins using various combinations of chromatography including the purification process of the present invention.

### DETAILED DESCRIPTION OF THE INVENTION

Unless defined otherwise, all technical and scientific terms used herein have the same meanings as appreciated by those skilled in the field to which the present invention pertains. In general, the nomenclature used herein is well-known in the art and is ordinarily used.

The present invention is based on the finding that, when a botulinum toxin culture solution is purified by multimodal chromatography and cation exchange chromatography in this order, botulinum toxin can be purified with much higher purity compared to botulinum toxin purified by a conventional known method.

In one aspect, the present invention is related to a method of purifying botulinum toxin including (a) purifying botulinum toxin from a sample containing botulinum toxin using anion exchange chromatography (AEX), hydrophobic interaction chromatography (HIC), and size exclusion chromatography (SEC) and (b) purifying the botulinum toxin using cation exchange chromatography.

As used herein, the term "botulinum toxin" may encompass neurotoxins (NTXs) produced by *Clostridium* botulinum strains or variants thereof and variant, recombinant, hybrid and chimeric botulinum toxins. In the present invention, the recombinant botulinum toxin may have light and/or heavy chains that are produced by recombination with the species other than *Clostridium.* In the present invention, the botulinum toxin includes both botulinum toxin complexes (i.e., 300, 600 and 900 kDa complexes) and pure botulinum toxins (i.e., about 150 kDa neurotoxic molecules).

NTXs (7S) which are the main components of botulinum toxins are combined with nontoxic components in cultures or in foods to form large complexes (Oguma et al., "Structure and function of Clostridium botulinum progenitor toxin.", J. Toxicology: Toxin Reviews, 18:17-34, 1999). Type A strain that produces botulinum toxin serotype A produces three forms of progenitor toxins designated as LL (19S, 900 kDa), L (16S, 500 kDa) and M (12S, 300 kDa) toxins, all of which are considered to be fully activated, while type B, C, and D strains produce two forms, L and M. In addition, type E, F and G produce only a single form of toxin; types E and F produce M toxin, and type G produces L toxin. M toxin consists of a NTX (7S, 150 kDa) and a nontoxic component having no hemagglutinin (HA) activity, which is described as non-toxic non-HA (NTNH).

In the present invention, the botulinum toxin may be selected from the group consisting of serotypes A, B, C, D, E, F, and G, but is not limited thereto.

In the present invention, the sample containing botulinum toxin in step (a) is used to include, without limitation, samples containing one or more botulinum toxins and impurities. For example, the sample may be a culture solution of a *Clostridium* botulinum strain, a lysate thereof, or a concentrate or precipitate thereof. In another example, the sample may include an initial or intermediate process solution or result that may be produced in the process of the present invention or another process of the invention. Alternatively, the sample may include, but is not limited to, a recombinant sample containing a recombinant botulinum toxin.

In the present invention, the sample containing the recombinant botulinum toxin in step (a) may be filtered and injected into a chromatographic matrix or resin. In the present invention, filtration in step (a) may be performed using a filter of 0.01 to 2 µm, preferably 0.05 to 1.5 µm, and more preferably 0.1 to 1 µm, but is not limited thereto. In an example of the present invention, the sample may be filtered through a 0.2 µm filter before injection into a column packed with a chromatography resin, but it is not limited thereto.

In the present invention, the anion exchange chromatography, hydrophobic interaction chromatography, and size exclusion chromatography in step (a) may be performed in any order, or two or more of the chromatographies may be performed simultaneously using a multimodal chromatography resin.

As used herein, the term "multimodal" chromatography refers to chromatography in which the separation of the desired component, the botulinum toxin of the present invention, is based on one or more types of interaction between the components of the stationary phase and the mobile phase. Here, the term "multimodal" may be used interchangeably with "mixed mode".

In the present invention, step (a) is performed using a multimodal chromatography resin having functions of two or more chromatographies of anion exchange chromatography, hydrophobic interaction chromatography, and size exclusion chromatography. For example, step (a) may be performed using a resin with AEX and HIC functions, a resin with AEX and SEC functions, a resin with HIC and SEC functions, and/or a resin with all AEX, HIC, and SEC functions, but is not limited thereto.

In the present invention, step (a) may be performed using a multimodal resin having anion exchange chromatography, hydrophobic interaction chromatography, and size exclusion chromatography functions.

In the present invention, for example, step (a) comprises purifying the botulinum toxin-containing fraction, which purified using the multimodal resin having anion exchange chromatography and hydrophobic interaction chromatography functions, using size exclusion chromatography (SEC).

As used herein, the term "anion exchange chromatography" refers to a process of separating substances based on charge using an ion exchange resin containing a positively charged group such as a diethylaminoethyl group (DEAE). Various commercially available anion exchange chromatography resins may be used in the present invention. For example, the resins used in the anion exchange chromatography include diethylaminoethyl (DEAE), quaternary aminoethyl (QAE), and quaternary amine (Q) groups, and are preferably TQ650, HQ, XQ, QXL, Capto, and BigBeads resins, but are not limited thereto. For a more specific example, the anion exchange chromatography is described in Protein Purification Methods, A Practical Approach, Ed. Harris E L V, Angal S, IRL Press Oxford, England (1989); Protein Purification, Ed. Janson J C, Ryden L, VCH-Verlag, Weinheim, Germany (1989); Process Scale Bioseparations for the Biopharmaceutical Industry, Ed. Shukla A A, Etzel M R, Gadam S, CRC Press Taylor & Francis Group (2007), pages 188-196; Protein Purification Handbook, GE Healthcare 2007 (18-1132-29) and Protein Purification, Principles, High Resolution Methods and Applications (2nd Edition 1998), Ed. Janson J-C and Ryden L, but is not limited thereto.

In the present invention, the hydrophobic interaction chromatography is a method of separating target molecules depending on the degree of hydrophobicity. For example, the hydrophobic group such as phenyl, octyl or butyl of the target molecule is adhered through hydrophobic interaction with the HIC resin (stationary phase). In the present invention, various commercially available hydrophobic interaction chromatography resins may be used. For example, the hydrophobic interaction chromatography resin may include a hydrophobic moiety selected from an alkyl group, an aromatic group, and an ether, and more specifically, the alkyl group may include lower alkyl groups such as n-propyl, isopropyl, n-butyl, iso-butyl, and n-octyl, and the aromatic group may include substituted or unsubstituted phenyl, but is not limited thereto. In addition, the hydrophobic interaction chromatography resin may include a matrix including agarose, Sepharose (GE Healthcare), polystyrene, divinylbenzene, and combinations thereof, but is not limited thereto. For a more specific example, the hydrophobic interaction chromatography is described in Protein Purification Methods, A Practical Approach, Ed. Harris E L V, Angal S, IRL Press Oxford, England (1989) page 224, Protein Purification, Ed. Janson J C, Ryden L, VCH-Verlag, Weinheim, Germany (1989) pages 207-226, Process Scale Bioseparations for the Biopharmaceutical Industry, Ed. Shukla A A, Etzel M R, Gadam S, CRC Press Taylor & Francis Group (2007), pages 197-206, Hydrophobic Interaction and Reversed Phase Chromatography, Principles and Methods, GE Healthcare 2007 (11-0012-69), Protein Purification Handbook, GE Healthcare 2007 (18-1132-29) and Protein Purification, Principles, High Resolution Methods and Applications (2nd Edition 1998), Ed. Janson J-C and Ryden L, "Hydrophobic Interaction Chromatography, page 283, but is not limited thereto.

In the present invention, size exclusion chromatography, which is also known as molecular sieve chromatography, is a chromatography method that separates molecules depending on the size and molecular weight thereof. In the present invention, the size exclusion chromatography may be performed using various size exclusion chromatography resins known in the art or columns containing the same. For example, the size exclusion chromatography resin generally contains a polymer having fine porous beads, and materials are separated depending on the pore size of the beads. In the present invention, the polymer may be, for example, dextran, agarose, or polyacrylamide polymer, but is not limited thereto. In the present invention, the size exclusion chromatography may have a cutoff of about Mᵣ 10,000 to about Mᵣ 1,000,000, preferably about Mᵣ 100,000 to about Mᵣ 500,000, and most preferably about Mᵣ 400,000. In the present invention, the cutoff in size exclusion chromatography means that molecules with a size not less than predetermined Mᵣ are excluded.

In the present invention, various buffers may be used for the multimodal chromatography in step (a). For example, buffers that may be used for multimodal chromatography in step (a) may include at least one selected from the group consisting of sodium phosphate, sodium chloride, potassium phosphate, sodium citrate, histidine, tris(hydroxymethyl) aminomethane (Tris), and bis-tris, but is not limited thereto. In the present invention, the buffer for multimodal chromatography in step (a) may include an equilibration buffer, an elution buffer, and a washing buffer. In the present invention, the buffer for multimodal chromatography in step (a) may be selected and used at an appropriate concentration depending on the purpose (equilibration/elution or washing) or type of buffer.

In addition, it is obvious that various buffers known in the art may be used to perform respective chromatography.

In the present invention, the buffer may further contain at least one salt (e.g., NaCl) as needed, but is not limited thereto.

In the present invention, when a sodium phosphate buffer is used, it may be used at a concentration of 1 to 200 mM, preferably 10 to 150 mM, more preferably 25 to 100 mM, and most preferably 30 to 75 mM, but is not limited thereto.

In the present invention, the buffer may have pH 4 to 9, preferably pH 5 to 8, and more preferably pH 6 to 7.

In the present invention, the botulinum toxin in step (a) is obtained in the form of a FT (flow through) eluted from multi-mode chromatography as a fraction containing the botulinum toxin (flow through mode) or is obtained by eluting a fraction containing the botulinum toxin bound to a chromatographic resin (binding mode).

In the present invention, the botulinum toxin in step (a) may be obtained in a flow through mode in which FT (flow through) eluting from multimodal chromatography is obtained as a fraction containing the botulinum toxin. The terms "flow through" (FT), "flow through mode" or "flow through purification", as used interchangeably herein, refer to a purification method in which at least one target molecule (e.g. botulinum) contained in a biopharmaceutical formulation passes through a substance that binds to one or more impurities, and the target molecule usually does not bind to the substance (i.e., flows through the substance).

In the present invention, the cation exchange chromatography in step (b) refers to a process of separating substances based on charge using an ion exchange resin containing a negatively charged group. In the present invention, various commercially available cation exchange chromatography resins may be used, such as carboxymethyl (CM), sulfoethyl (SE), sulfopropyl (SP), phosphate (P) and sulfonate (S), and preferably HS, XS, or the like may be used, but are not limited thereto. For another example, the cation exchange chromatography is described in Protein Purification Methods, A Practical Approach, Ed. Harris E L V, Angal S, IRL Press Oxford, England (1989); Protein Purification, Ed. Janson J C, Ryden L, VCH-Verlag, Weinheim, Germany (1989); Process Scale Bioseparations for the Biopharmaceutical Industry, Ed. Shukla A A, Etzel M R, Gadam S, CRC Press Taylor & Francis Group (2007), pages 188-196; Protein Purification Handbook, GE Healthcare 2007 (18-1132-29) and Protein Purification, Principles, High Resolution Methods and Applications (2nd Edition 1998), Ed. Janson J-C and Ryden L, but is not limited thereto.

In the present invention, the cation exchange chromatography in step (b) may be performed in a binding mode in which the botulinum toxin is bound to a resin and then eluted therefrom. The term "binding mode," as used interchangeably herein refers to a separation method in which at least one target molecule (e.g., botulinum toxin) contained in a sample binds to a resin, whereas one or more impurities do not bind to the resin and pass therethrough (that is, flow through).

In the present invention, the botulinum toxin in step (b) may be bound to the cation exchange chromatography resin, and therefore, the method may further include eluting the botulinum toxin from the resin.

In the present invention, a cellulose ion exchange resin commercially available in the art may be used in the anion exchange chromatography of step (a) or the cation exchange chromatography of step (b). For example, DE23^{™}, DE32^{™}, DE52^{™}, CM-23^{™}, CM-32^{™}, and CM-52^{™} are commercially available from the producer [Cytiva], and SEPHADEX-based and cross-linked ion exchangers are also known. For example, DEAE-, QAE-, CM-, and SP-SEPHADEX and DEAE-, Q-, CM-, and S-SEPHAROSE and SEPHAROSE Fast Flow are all commercially available from the producer [Cytiva]. In addition, DEAE and CM-derivatized ethylene glycol-methacrylate copolymers (e.g., TOYOPEARL^{™} DEAE-650S or M and TOYOPEARL^{™} CM-650S or M) are commercially available from the producer [Tosoh Bioscience LLC, King of Prussia, PA.]

In the present invention, various buffer solutions may be used in the cation exchange chromatography in step (b). For example, the buffer that may be used in the cation exchange chromatography in step (b) includes at least one selected from the group consisting of citric acid, sodium citrate, sodium acetate, acetic acid, sodium succinate, succinic acid, 2-(N-morpholino)ethanesulfonic acid (MES), potassium hydrogen phthalate, and hydroxyethyl piperazine ethane sulfonic acid (HEPES), but is not limited thereto. In one embodiment of the present invention, a buffer may be prepared by mixing trisodium citrate with citric acid, but is not limited thereto.

In the present invention, the buffer for cation exchange chromatography in step (b) may include an equilibration buffer and an elution/washing buffer. In the present invention, the buffer for cation exchange chromatography in step (b) may be selected and used at an appropriate concentration depending on the purpose (equilibration, elution, or washing, etc.) or the type of buffer.

In the present invention, the buffer may further contain at least one salt (e.g., NaCl) as needed, but is not limited thereto.

In the present invention, when a sodium citrate buffer is used, it may be used at a concentration of 1 to 100 mM, preferably 5 to 50 mM, more preferably 10 to 30 mM, but is not limited thereto.

In the present invention, the buffer may have pH 1.0 to 9.0, preferably pH 2.0 to 7.0, more preferably pH 3.0 to 6.0, and most preferably pH 4.0 to 5.0.

In the present invention, in addition to the purification steps using chromatography of steps (a) and (b), the method may further include a typical process for purification of botulinum toxin.

In the present invention, the method may further include culturing *Clostridium* botulinum strains. The culturing *Clostridium* botulinum strains to produce botulinum toxin may be performed using conventional methods known in the art and may be performed using conventional media that may be used for culture.

As a non-limiting example, the medium for culturing *Clostridium* botulinum strains may include casein hydrolysate, yeast extract, glucose, and the like. As another example, a medium for culturing *Clostridium* botulinum strains may not contain an animal-derived product. In the present invention, the *Clostridium* botulinum strain may be cultured at a temperature of 25 to 40°C for 90 to 200 hours, preferably 100 to 150 hours, but is not limited thereto.

In the present invention, the method may further include filtration. In the present invention, the filtration may be performed in one or more steps among before step (a), between steps (a) and (b), or after step (b). In the present invention, the filtration may be performed before injection into a column packed with a chromatography resin.

In the present invention, the method may include filtration before step (a).

In the present invention, the filtration may be, for example, depth filtration (DF), microfiltration (MF), ultrafiltration (UF), sterile filtration, or a combination thereof, but is not limited thereto. The filtration of the present invention functions to remove or concentrate impurities, especially nucleic acid impurities, HCD, cell debris and endotoxins contained in the sample, and to replace buffers, and the like, but the functions of the filtration are not limited thereto.

As used herein, the term "depth filtration (DF)" refers to a process of removing particles (e.g., impurities) from a solution using a series of filters that gradually decrease in pore size, and as used herein, the term "depth filter" achieves filtration within the depth of the filtered material. The filter contains any fiber matrix forming complicated and tortuous mazes of flow channels and particle separation is possible by capture by or adsorption to the fiber matrix. The depth filter media most commonly used for bioprocessing cell culture broths and other feedstocks include cellulose fibers, filter aids such as DE, and positively charged resin binders. Unlike absolute filters, depth filter media retain particles through porous media, allowing retention of particles larger and smaller than the pore size. Particle retention may involve both size exclusion and adsorption through hydrophobic, ionic and other interactions. Commercially available depth filters include Millistak+ Pod depth filter system, XOHC media (Millipore Corporation), and Zeta Plus^{™} Depth Filter (3M Purification Inc.). In the present invention, depth filtration may be performed using two or more depth filters stacked in layers. In this case, commercially available Millistak+ mini DOHC (Millipore Corporation) and XOHC filters (Millipore Corporation) may be used.

In the present invention, the depth filter generally has a nominal pore size of 0.01 to 20 µm, preferably 0.05 to 8 µm, and most preferably 0.1 to 6 µm, and may include a porous depth filter medium having a plurality of graded layers to remove aggregated cell debris and colloidal particles having a size larger than the pore size or aggregated cellular biomass including particles smaller than the pore size.

Therefore, depth filtration in the present invention enables easy removal of impurities (e.g., nucleic acids), cell debris, endotoxins, and the like contained in a sample containing botulinum toxin.

As used herein, the term "microfiltration (MF)" or "ultrafiltration (UF)" refers to a process of fractioning a target solute (e.g., botulinum toxin) depending on the size and structure of the solute, which is a component of the mixed solution, through the pores of the membrane under a predetermined pressure. For example, the solution may be purified through a PS (polysulfone) membrane for separating substances with 0.1 µm or 750 kDa molecular weight cutoff (MWCO) under conditions of 5 to 40 psig and 4 to 60°C.

In general, microfiltration is a process that is performed before ultrafiltration and is used to separate 0.1 to 10 µm particles from a solution, and is generally used to separate polymers with a molecular weight of 1x10⁵ g/mol or more. In addition, microfiltration is used to remove sediment, protozoa, large bacteria, and the like. In the present invention, microfiltration may enable easy removal of polymers and cell debris. In general, the microfiltration is performed using a pressure pump or vacuum pump at a speed of 0.1 to 5 m/s, preferably 1 to 3 m/s, and a pressure of 50 to 600 kPa, preferably 100 to 400 kPa.

Ultrafiltration (UF) is used to separate particles with a size of 0.01 to 0.1 µm from a solution, and the particles generally correspond to polymers with a molecular weight of 1x10³ to 1x10⁵ Da. Ultrafiltration is used to remove proteins, endotoxins, viruses, silica, and the like, but is not limited thereto. When an ultrafiltration membrane for separating MWCO 100-300K substances is used for the present invention, impurities contained in a sample containing botulinum toxin may be removed and botulinum toxin may be concentrated.

As used herein, the term "sterile filtration" refers to a method of safely purifying solutions containing target substances (e.g., biologics, botulinum toxins, etc.) using microfiltration or membrane filters, instead of heating, irradiation, and chemical treatment. A microfilter with pores of 0.1 to 0.3 µm, preferably 0.15 to 0.25 µm, and most preferably 0.2 µm, is used to remove microorganisms that may be contained in the solution, a nanofilter with pores of 20 to 50 nm is used to remove and inactivate viruses. Likewise, purification is performed using membrane filters having a predetermined pore size and made of cellulose ester or PES (polyethersulfone) to remove microorganisms, viruses, etc.

In the present invention, the method of purifying botulinum toxin may further include depth filtration.

In the present invention, the depth filtration may be performed before step (a).

In the present invention, the depth filtration may be performed through filtration using a depth filtration filter and a sterile filtration filter.

In the present invention, the method may further include concentrating the filtered sample after the depth filtration. In the present invention, the concentration may be performed through ultrafiltration (UF). The concentration may be performed without limitation using various concentration methods or concentration cassettes known in the art. In the present invention, the concentration may be performed by concentrating the filtered sample by 2 to 100 times, 5 to 70 times, 10 to 50 times, or 20 to 40 times, but is not limited thereto.

In the present invention, the purification may further include ultrafiltration (UF).

In the present invention, the ultrafiltration may be performed before step (a).

In the present invention, the purification may further include diafiltration.

As used herein, the term "diafiltration (DF)" refers to a method of removing or obtaining components (e.g., particles) contained in a target material (solution) using a permeable filter depending on the molecular weight (molecular size) to improve purity of the target material.

In the present invention, diafiltration may be performed through ultrafiltration. The term "ultrafiltration/ diafiltration (UF/DF)" refers to diafiltration using the aforementioned ultrafiltration (UF), but is not limited thereto.

In the present invention, the filtration may be performed by tangential flow filtration (TFF).

In the present invention, the ultrafiltration may be performed by tangential flow filtration (TFF).

In the present invention, the tangential flow filtration (TFF), which is also known as "cross-flow filtration", refers to a method of filtration in which water and a sample passing through the membrane are disposed to flow at a right angle.

In the present invention, the filtration, for example, depth filtration and/or ultrafiltration, may be repeated one or more times before step (a), but is not limited thereto.

In the present invention, the method may further include filtration after steps (a) and (b). In general, the filtration after steps (a) and (b) may be performed for concentration or dilution to a concentration appropriate for storage and use, without being limited to these uses. For example, after the purification after steps (a) and (b), the protein concentration is 0.1 mg/mL to 5 mg/mL, preferably 0.5 mg/mL to 2.5 mg/mL, more preferably 1.0 mg/mL to 1.5 mg/mL, but is not limited thereto.

In the present invention, the method may not include acid precipitation before step (a). The "acid precipitation" refers to a step of precipitating toxins, for example, by adding an acid such as sulfuric acid or phosphoric acid.

In the present invention, the "pH" of a solution is determined by measuring acidity or alkalinity in relation to the ionization of a water sample. The pH of water is neutral, i.e. pH 7. Most pH ranges from 0 to 14. Solutions (less than pH 7) with a higher [H+] than water are acidic, whereas solutions (greater than pH 7) with a lower [H+] than water are basic or alkaline. pH may be measured using a pH meter. Buffer pH may be adjusted using acids or bases such as HCl or NaOH.

The botulinum toxin purified by the method including steps (a) and (b) of the present invention is pure botulinum toxin or botulinum toxin complex that has been separated or substantially separated from other proteins and impurities that may be incorporated into the botulinum toxin when the botulinum toxin is obtained from a culture or fermentation process.

The botulinum toxin purified by the method of the present invention exhibits a purity of 95% or more, preferably 97%, more preferably 98%, and most preferably 99% or more. In particular, in the embodiment of the present invention, when the toxin is purified by performing AEX+SEC+HIC combination multimodal chromatography (flow through mode), followed by cation exchange chromatography (binding mode), or by performing AEX+HIC combination multimodal chromatography, followed by sequentially performing SEC and CEX, the toxin could be obtained with high yield as well as high purity of 99% or higher.

In the present invention, a preferred method of purifying botulinum toxin includes:
(i) filtering a sample containing botulinum toxin;
(ii) purifying botulinum toxin from the filtered sample using multimodal chromatography including anion exchange chromatography (AEX), hydrophobic interaction chromatography (HIC) and size exclusion chromatography (SEC); and
(iii) purifying the botulinum toxin from the sample purified through multimodal chromatography using cation exchange chromatography.

In the present invention, a more preferred method of purifying botulinum toxin includes:
(i) depth-filtering a sample containing botulinum toxin;
(ii) ultrafiltering the depth-filtered sample;
(iii) purifying botulinum toxin from the filtered sample using multimodal chromatography including anion exchange chromatography (AEX), hydrophobic interaction chromatography (HIC) and size exclusion chromatography (SEC); and
(iv) purifying the botulinum toxin from the sample purified through multimodal chromatography using cation exchange chromatography.

In the present invention, another more preferred method of purifying botulinum toxin includes:
(i) filtering a sample containing botulinum toxin;
(ii) purifying botulinum toxin from the filtered sample using multimodal chromatography including anion exchange chromatography (AEX), hydrophobic interaction chromatography (HIC) and size exclusion chromatography (SEC);
(iii) purifying the botulinum toxin from the sample purified through multimodal chromatography using size exclusion chromatography (SEC); and
(iv) purifying the botulinum toxin from the sample purified through size exclusion chromatography using cation exchange chromatography.

In the present invention, a more preferred method of purifying botulinum toxin includes:
(i) depth-filtering a sample containing botulinum toxin;
(ii) ultrafiltering the depth-filtered sample;
(iii) purifying botulinum toxin from the filtered sample using multimodal chromatography including anion exchange chromatography (AEX), hydrophobic interaction chromatography (HIC) and size exclusion chromatography (SEC); and
(iv) purifying the botulinum toxin from the sample purified through multimodal chromatography using cation exchange chromatography.

In another aspect, the present invention is related to a composition containing botulinum toxin purified by the purification method of the present invention.

In the present invention, the composition containing the botulinum toxin may have a purity of about 95% or more, preferably about 97%, more preferably about 98%, and most preferably about 99% or more.

In the present invention, the composition containing the botulinum toxin contains about 2% or less, preferably about 1.5% or less, more preferably about 1% or less, more preferably about 0.5% or less, or about 0.4% or less of toxin impurities.

In the present invention, the composition containing the botulinum toxin exhibits an aggregation peak of about 2% or less, preferably about 1.5% or less, more preferably about 1% or less, and more preferably about 0.5% or less.

Hereinafter, the present invention will be described in more detail with reference to the following examples. However, it will be obvious to those skilled in the art that the following examples are provided only for illustration of the present invention, and should not be construed as limiting the scope of the present invention.

### Example 1: Main culture of Clostridium botulinum strains

*Clostridium* botulinum strain for the production of botulinum toxin was prepared by inoculating a medium containing 10 L of 2% casein hydrolysate, 1% yeast extract, and 1% glucose with *Clostridium* botulinum type A strain, followed by culturing at 35°C for 120 hours.

### Example 2: Filtration ((depth filtration and ultrafiltration/diafiltration (UF/DF)

A depth filtration filter and a sterile filtration filter were connected in series and then a culture solution was filtered at a flow rate of 300 mL/min while maintaining 0.35 bar or less to remove insoluble impurities and perform sterilization.

The filters used are as follows:
Depth Filtration Filter 3M^{™} Zeta Plus^{™} Encapsulated System Filter Capsule E1020FSA
Sterile Filtration Filter: Sartopore 2 MidiCaps

The depth-sterile filtered culture solution was concentrated 30-fold with a 100 kDa MWCO cassette and then the concentrated filtrate was diluted 2-fold with 50 mM sodium phosphate pH 6.5 and then concentrated 2-fold again with a 100 kDa MWCO cassette. This ultrafiltration was repeated 7 times to replace the buffer (diafiltration). Ultrafiltration was performed using tangential flow filtration (TFF).

### Example 3: 1st purification

In order to develop a better purification process, 1st purification was performed using the following chromatography. Before the 1st purification, whether or not the baseline of conductivity and UV 278 nm of the equipment (FPLC) was stabilized was determined.

### Example 3-1: Multimodal resin (AEX + HIC + SEC, Capto Core400) - FT mode

The column packed with a Capto Core 400 resin was equilibrated with equilibration buffer. The sample for which buffer replacement was completed through ultrafiltration was filtered through a 0.2 µm filter and injected at a linear speed of 60 to 150 cm/h. Then, 5 CV (column volume) or more of equilibration buffer were injected and the section that eluted in a flow through mode without binding to the resin was collected. 2 CV or more of washing buffer was allowed to flow to remove (elute) impurities bound to the column.

The buffers used are:
- Equilibration/elution buffer: 50 mM sodium phosphate pH 6.5
- Washing buffer: 50 mM sodium phosphate pH 6.5, 1 M NaCl

### Example 3-2: AEX resin (Toyopearl Super Q 650M) - binding mode

The column packed with a Toyopearl Q resin was equilibrated with equilibration buffer. The sample for which buffer replacement was completed through ultrafiltration was filtered through a 0.2 µm filter and then injected at 60 to 150 cm/h, and 5 CV or more of equilibration buffer was injected. 20 CV or more of washing buffer was allowed to flow at a gradient of 0 to 100% to sequentially elute toxins bound to the column. The section in which only toxins were eluted without impurities was identified and recovered.

The buffers used are:
- Equilibration/washing buffer: 50 mM sodium phosphate, pH 6.5
- Elution buffer: 50 mM sodium phosphate pH 6.5, 1 M NaCl

### Example 3-3: Multimodal resin (CEX + HIC, Capto MMC) - FT mode

The column packed with a Capto MMC resin was equilibrated with equilibration buffer. The sample for which buffer replacement was completed through ultrafiltration was diluted to pH 4.5 with 50 mM sodium citrate pH 4.0. A processing sample was injected at 60 to 150 cm/h and then 5 CV or more of equilibration buffer was injected. The section that eluted in a flow through mode without binding to the resin was collected. 10 CV or more of washing buffer was allowed to flow at a gradient of 0 to 100% to remove (elute) impurities bound to the column.

The buffers used are:
- Equilibration/washing buffer: 25 mM sodium phosphate, pH 4.5
- Elution buffer: 25 mM sodium phosphate pH 6.5, 1 M NaCl

### Example 3-4: Multimodal resin (AEX + HIC, Capto Adhere) - FT mode

The column packed with a Capto Adhere resin was equilibrated with equilibration buffer. The sample for which buffer replacement was completed through ultrafiltration was filtered through a 0.2 µm filter and injected at a linear speed of 60 to 150 cm/h. Then, 5 CV or more of equilibration buffer was injected and the section that eluted in a flow through mode without binding to the resin was collected. 10 CV or more of washing buffer was allowed to flow at a gradient of 0 to 100% to remove (elute) impurities bound to the column.

The buffers used are:
- Equilibration/elution buffer: 50 mM sodium phosphate pH 6.5
- Washing buffer: 50 mM sodium phosphate pH 6.5, 1 M NaCl

### Example 3-5: AEX resin (Toyopearl Super Q 650M) - FT mode

The column packed with a Toyopearl Q resin was equilibrated with equilibration buffer. The sample for which buffer replacement was completed through ultrafiltration was filtered through a 0.2 µm filter and injected at a linear speed of 60 to 150 cm/h. Then, 5 CV or more of equilibration buffer was injected and the section that eluted in a flow through mode without binding to the resin was collected. 10 CV or more of washing buffer was allowed to flow at a gradient of 0 to 100% to remove (elute) impurities bound to the column.

The buffers used are:
- Equilibration/elution buffer: 50 mM sodium phosphate, 10 mS/cm pH 6.5
- Washing buffer: 50 mM sodium phosphate pH 6.5, 1 M NaCl

### Example 3-6: HIC resin (Butyl Sepharose HP) - binding mode

The column packed with a butyl Sepharose HP resin was equilibrated with equilibration buffer. NaCl was added at 3M to the sample for which buffer replacement was completed through ultrafiltration, diluted 5-fold with equilibration buffer and filtered through a 0.2 µm filter. The sample was injected at a linear speed of 60 to 150 cm/h and 5 CV or more of equilibration buffer was injected. 10 CV or more of washing buffer was allowed to flow at a gradient of 0 to 100% to sequentially elute impurities bound to the column. Among the sections where toxins were eluted, the section containing the least impurities was identified and recovered.

The buffers used are:
- Equilibration/elution buffer: 50 mM sodium phosphate pH 6.5, 3M NaCl
- Washing buffer: 50 mM sodium phosphate pH 6.5

### Example 3-7: Multimodal resin (AEX + HIC, Capto Adhere) - FT mode + SEC resin (Superdex 75pg)

### 3-7-1: Multimodal resin (AEX + HIC, Capto Adhere)

The column packed with Capto Adhere resin was equilibrated with equilibration buffer. NaCl was added at 3M to the sample for which buffer replacement was completed through ultrafiltration, filtered through a 0.2 µm filter and then injected at a linear speed of 60 to 150 cm/h. 5 CV or more of equilibration buffer was injected. The section that eluted in a flow through mode without binding to the resin was collected. 10 CV or more of washing buffer was allowed to flow at a gradient of 0 to 100% to remove (elute) impurities bound to the column.

The buffers used are:
- Equilibration/elution buffer: 50 mM sodium phosphate pH 6.5
- Washing buffer: 50 mM sodium phosphate pH 6.5, 1 M NaCl

### 3-7-2: SEC resin (Superdex 75pg)

The eluate recovered from multimodal resin (AEX + HIC, Capto Adhere) was filtered through a 0.2 µm filter, a column packed with a Superdex 75 pg resin was equilibrated with 50 mM sodium phosphate (pH 6.5), and then the resulting filtrate was injected into the column at a flow rate of 30 cm/h. After completion of injection, equilibration/elution buffer was injected into the column in 5 mL portions until the UV value dropped to the baseline. Then, the fraction containing the toxin was recovered.

The buffers used is:
- Equilibration/elution buffer: 50 mM sodium phosphate pH 6.5

### Example 3-8: SEC resin (Superdex 200pg)

The sample for which buffer replacement was completed through ultrafiltration was filtered through a 0.2 µm filter, a column packed with a Superdex 200 pg resin was equilibrated with 50 mM sodium phosphate (pH 6.5) and then the resulting filtrate was injected into the column at a flow rate of 30 cm/h. After completion of injection, equilibration/elution buffer was injected in 5 mL portions until the UV value dropped to the baseline. Then, the fraction containing the toxin was recovered.

The buffer used is:
- Equilibration/elution buffer: 50 mM sodium phosphate pH 6.5

### Example 4-1: 2nd purification (CEX, Toyopearl SP-650S) - Binding mode

The samples primarily purified under the conditions of Examples 3-1 to 3-8 were diluted to pH 4.5 with 50 mM sodium citrate pH 4.0. Equilibration buffer was allowed to flow through a column packed with Toyopearl SP-650S resin to perform equilibration. The diluted solution was injected at 60 to 150 cm/h, and 5 CV or more of equilibrium buffer was injected. 10 CV or more of washing buffer was allowed to flow at a gradient of 0 to 100% to sequentially elute and recover toxins bound to the column.

The buffers used are:
- Equilibration/washing buffer: 20 mM sodium citrate pH 4.5
- Elution buffer: 20 mM sodium citrate pH 4.5, 1 M NaCl

### Example 5: Comparison with the latest purification process

To compare the effects between the botulinum toxin purification process according to the present invention and the latest botulinum toxin purification process, the purification process of Korea Patent No. 10-2512757 registered in 2023 was used as a control group.

### Example 5-1: 1st purification (AEX, Toyopearl NH2-750F) - Binding mode

The sample for which buffer replacement was completed through ultrafiltration was filtered through a 0.2 µm filter and then injected into a column packed with a Toyopearl NH2-750F resin at 46 cm/h. After injection, the column was washed with 5 CV of 50 mM sodium phosphate buffer pH 6.5 to equilibrate the UV value and conductivity. After washing, 2 mL of fractions were collected while eluting at 60% using 50 mM sodium phosphate buffer pH 6.5 and 1 M sodium chloride buffer, and were pooled based on SDS-PAGE results.

The buffers used are:
- Equilibration/washing buffer: 50 mM sodium phosphate pH 6.5
- Elution buffer: 50 mM sodium phosphate, 1 M NaCl pH 6.5

### Example 5-2: CEX (Toyopearl Sulfate-650F)-Binding mode

25 mM citrate buffer pH 5.5 was added to the eluate recovered in Example 5-1 to adjust the pH to 5.5 ± 0.1 and conductivity to 7.0 ± 0.5 mS/cm. Foreign substances were removed by filtration through a 0.22 µm bottle top filter. The filtered toxin solution was injected into a column filled with Sulfate-650F, a cation exchange resin, at a linear speed of 46 cm/hr. After injection, the result was washed with 25 mM citrate buffer pH 5.5 at 4.5 CV to equilibrate UV and conductivity. After washing, 2 mL of fractions were collected while eluting with 25 mM citrate buffer pH 5.5 and 1 M sodium chloride buffer at 14% and pooled based on the SDS-PAGE results.

The buffers used are:
- Equilibration/washing buffer: 25 mM sodium citrate pH 5.5
- Elution buffer: 25 mM sodium citrate, 1 M NaCl pH 5.5

### Example 6: Filtration and Storage

The concentration of the purified botulinum toxin protein was measured. When the concentration was 1.5 mg/mL or more, the protein was diluted to a concentration of 1.0 mg/mL to 3.0 mg/mL with 20 mM sodium citrate pH 4.5 buffer and then filtered through a 0.2 µm filter. The purified toxin was aliquoted and stored in a freezer at -70°C.

### Example 7: Results

### Example 7-1: Toxin yield

The yield of botulinum toxin obtained through various 1st purification processes and 2nd purification processes using CEX was measured. As can be seen from FIGS. 2 and 3, when 2nd purification (CEX) was performed after 1st purification (multimodal, AEX+HIC+SEC) of Example 3-1, a high yield of 29.25% was obtained. In addition, it can be seen that a high yield of 22.47% was obtained even when multimodal chromatography (AEX+HIC) and SEC were sequentially performed in the 1st purification.

When AEX (binding mode), a common method known in the art, and CEX were sequentially performed, a low yield of 12.47% was obtained. Other 1st purification chromatography had multimodal chromatography (CEX+HIC: 27.29%), AEX+HIC (27.74%), AEX-FT (32.74%), SEC (17.77%), or HIC (19.06%).

The purification process of Korean Patent No. 2512757, which is the latest purification process, exhibited a slightly high yield, but exhibited a very low purity below the reference level, making it unsuitable regardless of the yield, as will be described later. Therefore, it can be seen that the yield of botulinum toxin from the purification process of the present invention is similar to or higher than those of various recently developed purification processes.

### Example 7-2: Confirmation of purity of botulinum toxin (SDS-PAGE)

To determine whether or not the purified botulinum toxin sample contained impurities, SDS-PAGE was performed on each sample. As can be seen from FIGS. 4 and 5, impurity bands were observed in other 1st purification processes excluding 1st purification of Example 3-1 (multimode, AEX + HIC + SEC), 1st purification of Example 3-6 (HIC-Butyl), and 1st purification of Example 3-7 (multiple mode (AEX+HIC) and then SEC). The toxin bands itself were not detected in the peaks described in the prior literature regarding the latest purification processes and impurity bands were observed in the peaks containing the toxin.

### Example 7-3: Confirmation of purity of botulinum toxin (SEC-HPLC) and agglutination

In order to determine more accurate purity, SEC-HPLC was performed using TSK G-4000 to measure the toxin purity of the final purified sample.

As a result, as can be seen from FIGS. 6 to 9, when 1st purification (multiple mode, AEX + HIC + SEC) of Example 3-1 and 2nd purification of CEX were performed, a much higher purity of 99.72% was obtained. When 1st purification (multiple mode (AEX+HIC) and then SEC) of Example 3-7 and 2nd purification of CEX were performed, a remarkably high purity of 99.19% was obtained. In particular, the purification including the 1st process of Examples 3-1 and 3-7 have a very low impurity content and aggregation compared to other processes, which indicates that the botulinum toxin purification method according to the present invention has remarkably higher purity and stability than conventional methods.

In addition, Examples 3-2 to 3-6 have a low purity of about 94 to 96%, compared to Example 3-1, an impurity peak of 1 to 3% and at least 8 times higher aggregation, indicating problems of stability. On the other hand, the purification process (Example 5) of the prior literature has a very low purity of about 69%.

As described above, considering the strong toxicity of botulinum toxin, the purity and stability of the toxin after purification are very important factors. However, when the purity is increased to about 99%, the yield inevitably drops drastically. Therefore, most research has focused on increasing the yield at a purity of 95%.

The results of the present invention show that, when a purification process including 1st purification including multimodal chromatography including SEC, or sequentially performing multimodal chromatography and SEC, and 2nd purification of CEX, beyond the limits of the prior art, botulinum toxin with excellent stability can be obtained with a remarkably high purity of 99% or more and a yield higher than that of the prior art.

### Industrial applicability

The method of purifying botulinum toxin of the present invention is useful for the production of botulinum toxin because the botulinum toxin can be purified with an ultrahigh purity of about 99% or higher and a higher yield than that of a purification method generally used in the art.

Although specific configurations of the present invention have been described in detail, those skilled in the art will appreciate that this detailed description is provided as preferred embodiments for illustrative purposes, and should not be construed as limiting the scope of the present invention. Therefore, the substantial scope of the present invention is defined by the accompanying filed claims and equivalents thereto.

## Claims

1. A method of purifying botulinum toxin comprising:
(a) purifying botulinum toxin from a sample containing botulinum toxin using anion exchange chromatography (AEX), hydrophobic interaction chromatography (HIC), and size exclusion chromatography (SEC); and
(b) purifying the botulinum toxin using cation exchange chromatography.

2. The method according to claim 1, wherein step (a) is performed using a multimodal resin having functions of anion exchange chromatography, hydrophobic interaction chromatography, and size exclusion chromatography.

3. The method according to claim 1, wherein step (a) comprises purifying a fraction containing botulinum toxin purified using the multimodal resin having functions of anion exchange chromatography and hydrophobic interaction chromatography, using size exclusion chromatography.

4. The method according to claim 2 or 3, wherein the botulinum toxin in the multimodal chromatography in step (a) flows through the multimodal chromatography resin, rather than binding to the multimodal chromatography resin.

5. The method according to claim 1, wherein the botulinum toxin in the cation exchange chromatography in step (b) binds to a cation exchange resin and is then eluted.

6. The method according to claim 1, wherein the sample containing the botulinum toxin in step (a) is a culture solution of a *Clostridium* botulinum strain, a lysate thereof, or a precipitate thereof.

7. The method according to claim 1, further comprising filtration.

8. The method according to claim 1, further comprising:
(i) depth-filtering a sample containing botulinum toxin; and
(ii) ultrafiltering the depth-filtered sample.

9. The method according to claim 8, wherein step (ii) is performed by tangential flow filtration (TFF).

10. The method according to claim 1, wherein a finally purified botulinum toxin has a purity of 99% or higher.

11. A composition comprising botulinum toxin purified by the method according to claim 1.
